Europäisches Patentamt

European Patent Office

Office européen des brevets

(19)

(11) Veröffentlichungsnummer: **0 160 172**
**B1**

# EUROPÄISCHE PATENTSCHRIFT

(12)

(45) Veröffentlichungstag der Patentschrift:
27.09.89

(21) Anmeldenummer: 85102035.4

(22) Anmeldetag: 23.02.85

(51) Int. Cl.⁴: **G 01 M 15/00**

(54) Verfahren und Vorrichtung zum Bestimmen des Kraftstoff-Luftverhältnisses von Ottomotoren.

(30) Priorität: 31.03.84 DE 3412166

(43) Veröffentlichungstag der Anmeldung:
06.11.85 Patentblatt 85/45

(45) Bekanntmachung des Hinweises auf die Patenterteilung:
27.09.89 Patentblatt 89/39

(84) Benannte Vertragsstaaten:
AT BE CH DE FR GB IT LI LU NL SE

(56) Entgegenhaltungen:
EP-A- 0 071 474
DE-A- 3 006 525
DE-C- 1 598 827

(73) Patentinhaber: PIERBURG GMBH, Leuschstrasse 1,
D-4040 Neuss 1 (DE)

(72) Erfinder: Kampelmühler, Franz, Dr. Ing.,
Schillerstrasse 69, D-7146 Tamm (DE)

(74) Vertreter: Bergen, Klaus, Dipl.-Ing. et al, Patentanwälte
Dr.-Ing. Reimar König Dipl.-Ing. Klaus Bergen
Wilhelm-Tell-Strasse 14 Postfach 260162,
D-4000 Düsseldorf 1 (DE)

## Beschreibung

Die Erfindung betrifft ein Einkomponenten-Abgasmeßverfahren zum Bestimmen der Luftzahl bzw. Gemischzusammensetzung insbesondere von Ottomotoren, bei dem der Abgasstrom gekühlt, vom Kondensat befreit, gereinigt und anschließend mit einem gereinigten Luftstrom in einem konstanten Verhältnis gemischt wird, sowie eine zum Durchführen dieses Verfahrens geeignete Vorrichtung.

Aus der deutschen Patentschrift 1 598 827 ist ein konstantes Mengenverhältnis zwischen Abgas und Frischluft als wesentliche Voraussetzung für eine Gasentnahmesonde zur Kohlenmonoxydanalyse bei Ottomotoren an sich bekannt. Zwar wird dort ein konstantes Mengenverhältnis der Ströme als wesentliche Meßvoraussetzung genannt, jedoch bleibt völlig offen, wie dieses konstante Mischungsverhältnis eingestellt werden könnte. Außerdem läßt sich dort nur eine mit Fehlern behaftete Kohlenmonoxyd-Analyse durchführen; auf eine völlige Nachoxydation des mit Luft verdünnten Motorabgases kommt es nicht an. Die Konzentration von Kohlenmonoxyd im Motorabgas ist auch nur solange ein Maß für die Luftzahl, als der Motor im Luftmangelbereich und ohne Verbrennungsaussetzer betrieben wird.

Für die ordnungsgemäße Funktion von Vergaser- und Einspritzmotoren ist die korrekte Einstellung des Luft/Kraftstoffverhältnisses im Leerlauf eine notwendige Voraussetzung. Dieses Luft/Kraftstoffverhältnis wird durch die Luftzahl Lambda des Gemisches ausgedrückt, die sich als Quotient aus der tatsächlich verbrauchten Luftmenge und der für die stöchiometrische Verbrennung des Kraftstoffes notwendigen Luftmenge ergibt. Abweichungen von der vom Fahrzeughersteller vorgeschriebenen Einstellung sind häufig für eine erhöhte Umweltbelastung und einen unbefriedigenden Motorbetrieb verantwortlich.

Neben dem direkten Messen des Luft- und des Kraftstoffdurchsatzes, beispielsweise mittels Drehkolben- und Kammergaszählern, Meßgläsern, Verdränger- und Turbinenzählern, ist es weiterhin bekannt, die Luftzahl aus der Abgaszusammensetzung unter Berücksichtigung der Abhängigkeiten der verschiedenen Abgaskomponenten wie Kohlenmonoxyd, Kohlendioxyd, Sauerstoff, Wasserstoff und Kohlenwasserstoff zu bestimmen. Die Mehrkomponentenmessverfahren ermöglichen zwar eine ausreichend genaue Analyse der Abgaszusammensetzung, sie erfordern aber andererseits einen sehr hohen meßtechnischen und finanziellen Aufwand. Neben den hohen Anschaffungs- und Betriebskosten spricht gegen den Einsatz derartiger Meßverfahren insbesondere in Werkstätten noch die Notwendigkeit, daß es einer teilweise durch Mikroprozessoren unterstützten rechnerischen Auswertung der Meßwerte bedarf, was häufig das Bedienungspersonal überfordert.

Um den Meßaufwand zu reduzieren, ist es aus «Motor Technische Zeitschrift MTZ 37», Heft 3, 1976, Seiten 75/77, bekannt, lediglich die Abgasbestandteile Kohlenmonoxyd, Kohlendioxyd und Kohlenwasserstoff zu messen, wodurch sich aber die Genauigkeitsanforderungen an die Analyse der Einzelkomponenten erhöhen, was aber praktisch wegen der Abhängigkeiten der Einzelkomponenten untereinander nicht zu erreichen ist.

Um den meßtechnischen Aufwand bei der Abgasanalyse möglichst klein zu halten, ist es somit wünschenswert, bei einer zumutbaren Fehlerquote möglichst nur mit einer Abgaskomponente auszukommen. Da sich jedoch beim Betrieb eines Verbrennungsmotors der Luftzahlbereich von 0,7 bis 1,3 bewegt, d.h. zwischen einem reichen und einem mageren Gemischbereich schwankt, läßt sich das nur schwer realisieren. Der Gedanke, die Luftzahl aus der Abgasanalyse mittels Einkomponentenmessung zu gewinnen, ist an sich nicht neu, zumal davon auszugehen ist, daß Analysewerte für den im Abgas verbleibenden Restsauerstoff oder die Konzentration des Verbrennungsproduktes, nämlich Kohlendioxyd, ein direktes Maß für die Zusammensetzung des Gemisches darstellen. Zur Nachreaktion werden dabei allerdings Oxydations-Katalysatoren benötigt, die relativ teuer und beim Betrieb mit verbleitem Kraftstoff auch nur von kurzer Lebensdauer sind.

Es ist davon auszugehen, daß für eine erfolgreiche Anwendung eines Einkomponenten-Luftzahlmeßverfahrens eine vollständige Oxydation des Abgases sowie zum Analysieren unterstöchiometrischer Gemische in einem Lambdabereich kleiner als 1 das Beimischen einer gewissen Luft- oder Sauerstoff-Menge erforderlich ist. Damit wird die vollständige Oxydation der Abgase ermöglicht. Hierbei legt die untere Meßbereichsgrenze den erforderlichen Verdünnungsgrad der Abgase fest. Den Abgasen muß nämlich soviel Luft beigemengt werden, daß sie an der unteren Meßbereichsgrenze gerade noch vollständig oxydieren können. Das eine vollständige Oxydation des Abgases erfordernde Einkomponenten-Meßverfahren ist somit abhängig von einem konstanten, das vollständige Oxydieren gerade gewährleistenden Mengenverhältnis zwischen dem Abgas- und dem Frischluftstrom.

Durch die deutsche Offenlegungsschrift 3 006 525 ist die vollständige Oxydation eines sich aus einem Kohlenwasserstoff-Heizbrennstoff und Sauerstoff zusammensetzenden Gasgemischs bei einem Gerät zum Messen des Brennstoff-Luftverhältnisses eines Verbrennungssystems bekanntgeworden. Dieses bekannte Gerät ist außerordentlich aufwendig und erfordert insbesondere zwei katalytische Reaktoren, die relativ teuer und beim Betrieb mit verbleitem Kraftstoff nur von kurzer Lebensdauer sind. Die katalytischen Reaktoren sind unterschiedlich groß, wobei in dem größeren Reaktor ein Gemisch aus Heizbrennstoff und Luft verbrannt wird. Nach dem Oxydationsprozeß des Heizbrennstoff-Luft-Gemisches bleibt ein Sauerstoffüberschuß übrig, der mit dem Gemischstrom in den zweiten Katalysator geleitet und zur vollständigen Oxydation mit einem aus der Auspuffleitung einer Brennkraftmaschine entnommenen Abgasstrom vermischt wird. In diesem zweiten Katalysator werden sämtliche im Auspuffgas verbliebenen oxidierbaren Produkte zu Kohlendioxyd oder Wasser oxidiert. Sowohl der dem ersten Reaktor zuströmende Heizbrennstoffstrom als auch der Luftstrom werden mittels Regelventilen auf einen von dem ausgewählten Brennstoff-Luft-Verhältnis bestimmten Druck vorab fest eingestellt. Beide Ströme vermischen sich nach dem Passieren von Drosselventilen, noch bevor sie in den Reaktor einströmen. Abgesehen von den äußerst aufwendigen Oxidations-Katalysatoren ist als besonders nachteilig anzusehen, daß die Zustände der den Katalysatoren zugeführten Heizbrennstoff- und Luft- bzw. Abgasströme hinter den Druckregelventilen für das anschließende Mischen der Ströme unberücksichtigt bleiben; konstante Mengenverhältnisse lassen sich auf diese Weise nicht erreichen.

Der Erfindung liegt die Aufgabe zugrunde, die Nachteile der bekannten Verfahren und Geräte zu beseitigen und insbesondere ein konstantes Mengenverhältnis der miteinander zu vermischenden Ströme zu ermöglichen.

Die Aufgabe wird für das Verfahren erfindungsgemäß dadurch gelöst, daß die beiden Ströme druck- oder mengenabhängig sowie ventilgesteuert/-geregelt miteinander vermischt werden, die Konzentration der Meßwert-/Abgaskomponenten nach dem Mischen in Relation zu einem für jeden Strom vor dem Mischen feststehenden Ist-Wert gesetzt und bei Abweichungen vom Ist-Wert entweder ein Druckunterschied zwischen den beiden Strömen ausgeglichen oder die zugeführte Menge eines Stromes geändert und das Gemisch vollständig oxidiert und der Gehalt der zu bestimmenden Meßkomponenten in dem vollständig oxidierten Abgas-/Luftgemisch ermittelt wird. Mit diesen Maßnahmen läßt sich innerhalb kürzester Zeit, in Bruchteilen von Sekunden, eine Luftzahländerung im Abgas bei einem innerhalb eines mageren und eines reichen Gemischs liegenden nutzbaren Meßbereich mit größter Genauigkeit feststellen. Ohne daß aufwendige Vorrichtungen, wie insbesondere Katalysatoren, erforderlich sind, lassen sich auch Messungen außerhalb des Leerlaufbereichs durchführen, wie z.B. Messungen auf einem Fahrzeug- und einem Motorprüfstand. Ein konstantes, die Voraussetzung für eine vollständige Oxydation des Abgases und genaue Messungen bildendes Mengenverhältnis von Abgas- und Frischluftstrom gewährleistet hierbei das druck- oder mengenabhängige Vermischen der beiden Ströme. Entweder werden Druckverluste in den Zuleitungen berücksichtigt und danach gezielt ausgeglichen, oder es wird der gewünschte konstante Verdünnungsgrad d.h. das konstante Mischverhältnis abhängig vom jeweiligen, sich ändernden Zustand der Abgasströme eingestellt.

Vorzugsweise kann eine kleine Teilmenge des Gemischs vollständig oxidiert und analysiert werden. Das Aufteilen des Gemisches erlaubt ein schnelles Aufheizen der zum Messen benötigten Gemischmenge auf die zum Nachverbrennen erforderliche Temperatur von über 700 °C, was bei einem geringen Förderstrom einfacher ist als bei einem größeren Förderstrom; die einzubringende Heizleistung reduziert sich gleichermaßen. Insbesondere, da mit einer stets gleichbleibenden Kraftstoffzusammensetzung für Super- und Normalbenzin nicht gerechnet werden kann, ist wegen der Unempfindlichkeit gegenüber geänderten Kraftstoffzusammensetzungen das Sauerstoff-Meßverfahren vorzuziehen.

Änderungen der Kraftstoffzusammensetzung, wie sie durch das Mischen von unterschiedlichen Benzinfraktionen entstehen, haben nämlich einen Einfluß auf das Luftzahlmeßergebnis. Eine Zunahme des Kohlenstoff-Gehaltes im Kraftstoff ruft auch eine Zunahme der Kohlendioxyd-Konzentration im völlig oxidierten Abgas hervor, womit sich die Zuordnung der Kohlendioxyd-Konzentration zum Luftzahlwert verschiebt. Bei der Sauerstoffmessung wird hingegen bei einer Zunahme des Kohlenstoff-Gehaltes im Kraftstoff mehr Sauerstoff aus der Luft für die Kohlendioxyd-Bildung verbraucht. Dieser Mehrverbrauch wird aber zu einem Teil durch einen Sauerstoff-Minderverbrauch für die Wasserentstehung kompensiert.

Für eine Vorrichtung zum Durchführen des erfindungsgemäßen Verfahrens wird die Aufgabe erfindungsgemäß dadurch gelöst, daß in einer Abgasleitung ein Kühler, ein Wasserabscheider und ein Feinfilter sowie in einer Luftleitung ein Feinfilter angeordnet sind, daß sich daran in jeder Leitung eine Dosierdüse anschließt, die mit einer gemeinsamen, zu einem nachgeschalteten Verbrenner und einem Gassensor oder einer Sauerstoffsonde führenden Ausgangsleitung verbunden ist und daß ein Differenzdruckfühler mittels einer den Dosierdüsen vorgeordneten Zwischenleitung an die Luft- und die Abgasleitung angeschlossen und mit einem gesteuerten Drosselventil in einer Leitung, vorzugsweise in der Luftleitung gekoppelt ist. Mit dem Drosselventil läßt sich der Zustand der beiden Förderströme vor den beiden Dosierdüsen gleichstellen. Da die Temperatur vor den beiden Düsen gleich ist, hängt dieser Zustand nur vom Druck ab. Die vorhandenen Druckunterschiede erfaßt der vor den beiden Dosierdüsen angeordnete und steuerungs-regeltechnisch mit dem Drosselventil verbundene Differenzdruckfühler, so daß sich mit dem Drosselventil unterschiedliche Druckverluste in den Zuleitungen und den Filtern gezielt ausgleichen lassen.

Das exakte Einhalten des Differenzdruckes vor der Mischstufe ist ein maßgebliches Kriterium für die Langzeitkonstanz des Gerätes. Es muß versucht werden, die Schwankungen möglichst gering zu halten, beispielsweise kleiner +/− 10 hPa; Einflußgrößen für den Differenzdruck sind der Druck im Auspuffsystem, die Leitungslänge der Abgaszuführung und die Filterverschmutzung in der Gasaufbereitung. Das läßt sich bevorzugt dann erreichen, wenn der Differenzdruckfühler vor den Dosierdüsen mit einem in der Luftleitung angeordneten, motorbetriebenen Ventil gekoppelt ist. Beispielsweise kann ein an sich bekanntes Nadelventil von einem Miniaturmotor, d.h. von einem kleinbauenden Motor mit geringer Leistung, angetrieben und mit einer Elektronik an die zentrale Steuer- bzw. Anzeigeeinheit angeschlossen sein. Aus der Stellung des Ventils lassen sich auch Statusmeldungen für den Grad der Filterverschmutzung und für den Abgasüberdruck ableiten.

Eine in der gemeinsamen Ausgangsleitung als Saugpumpe eingesetzte Vakuumpumpe erzeugt den zum überkritischen Durchströmen der Düsen nötigen Unterdruck. Da beide Förderströme gemeinsam angesaugt werden, reicht eine einzige Pumpe aus.

Für eine Vorrichtung zum Durchführen des erfindungsgemäßen Verfahrens läßt sich die Aufgabe erfindungsgemäß auch dadurch lösen, daß in einer Abgasleitung ein Kühler, ein Wasserabscheider und ein Feinfilter sowie in einer Luftleitung ein Feinfilter angeordnet sind, daß sich daran in jeder Luftleitung ein Drei-Zwei-Wege-Magnetventil anschließt, das mit einer gemeinsamen, zu einem nachgeschalteten Verbrenner und einem Gassensor oder einer Sauerstoffsonde führenden Ausgangsleitung verbunden ist, daß jedes Magnetventil mittels einer Zweigleitung am Ventilausgang des jeweils anderen Magnetventils mit der Luft- bzw. Abgasleitung verbunden ist und daß einem, vorzugsweise dem in der Luftleitung angeordneten Magnetventil ein Drossel- oder Motorventil zugeordnet ist. Durch dieses Vermischen der beiden Gasströme quasi über Kreuz, während der Motor mit konstanter Luftzahl läuft, läßt sich für den gewünschten Verdünnungsgrad eine feste Zuordnung zwischen der Sauerstoff- oder Kohlendioxyd-Konzentrationsanzeige vor und nach dem Mischen der Gasströme ermitteln. Bei Abweichungen von dieser Zuordnung, d.h. vom Istwert, läßt sich der Istwert und somit der gewünschte Verdünnungsgrad durch Verstellen des Drosselventils wieder ausgleichen, indem entweder mehr oder weniger Luft mit dem Abgasstrom vermischt wird. Hiermit läßt sich vorteilhaft ein vollautomatisches Kalibrieren/Dosieren des Mischungsverhältnisses von Luft zu Abgas verwirklichen.

Der Nachbrenner kann als elektrisch beheizter Konverter mit gegebenenfalls eingebautem oder extern angeordnetem Temperaturfühler ausgebildet sein. Der Temperaturfühler signalisiert hierbei die Betriebsbereitschaft des Nachbrenners.

Dem Nachbrenner können ein Druchflußmesser und in einer Nebenleitung ein Bypassventil zugeordnet sein, so daß sich die gewünschte Teilmenge durch den Nachbrenner leiten läßt, während die Restmenge über das Bypassventil und die Nebenleitung abströmt.

Eine im Ausgang, vorteilhaft direkt in den Nachbrenner eingebaute Sauerstoffsonde, vorzugsweise aus einer bei höheren Temperaturen ansprechenden Oxydkeramik, wie vorteilhaft ein Zirkoniumdioxyd-Sauerstoffsensor, ermöglicht eine kompakte Bauweise des Meßgerätes und benötigt insbesondere keinen nachgeschalteten Gasanalysator.

Mit einem am Nachbrenner angeordneten Ventilator, der z.B. am Gehäuse befestigt sein kann, läßt sich ein ausgeglichenes Temperaturniveau erreichen, womit Abweichungen der Elektronik in zulässigen Grenzen gehalten werden. Die Meßgenauigkeit eines Zirkoniumdioxyd-Sauerstoffsensors hängt nämlich nicht so sehr von der Absoluttemperatur, wie vielmehr vom Temperaturgradienten ab; das bedeutet, daß Temperaturschwankungen und damit eine inhomogene Temperaturverteilung im Sensor möglichst klein zu halten sind. Hierzu kann weiterhin auch eine Regel- und Steuereinheit zum Unterdrücken von Spannungsspitzen bei der Regelung der Heizwicklung des Nachbrenners beitragen.

Sofern die aufbereiteten Abgase nach dem Verlassen des Nachbrenners einem separaten Sauerstoff- oder Kohlendioxyd-Analysator zuströmen, befindet sich zwischen dem Nachbrenner und dem Gassensor, in dem auch gleichzeitig der Temperaturfühler angeordnet sein kann, noch ein Kühler und ein Wasserabscheider, damit der bei der Nachreaktion der unverbrannten Kohlenwasserstoffe wieder entstehende Wasserdampf aufbereitet werden kann.

Bei einem vorzugsweise aus einem Keramikgehäuse und einer isolierenden Umhüllung bestehenden Nachbrenner lassen sich sowohl die zum Erreichen der benötigten Arbeitstemperatur erforderlichen Heizleistungen als auch der Wärmeübergang auf den Sensor verbessern. Das beispielsweise zur Wärmeisolation von einem Isoliermaterial und einem Metallmantel umhüllte Keramikgehäuse ermöglicht zudem einen fertigungstechnisch günstigen Nachbrenner.

Die Meßvorrichtung läßt sich manuell regeln oder über eine zentrale Schalt-, Steuer- und Anzeigeeinheit betreiben, die gegebenenfalls extern angeordnet sein kann.

Die Erfindung wird nachfolgend anhand der in der Zeichnung dargestellten Ausführungsbeispiele des näheren erläutert. In der Zeichnung zeigen:

Fig. 1 ein Flußschema einer ersten Meßeinheit, symbolisch dargestellt;

Fig. 2 einen Querschnitt durch einen elektrisch beheizten Nachbrenner; und

Fig. 3 ein Flußschema einer zweiten Meßeinheit, symbolisch dargestellt.

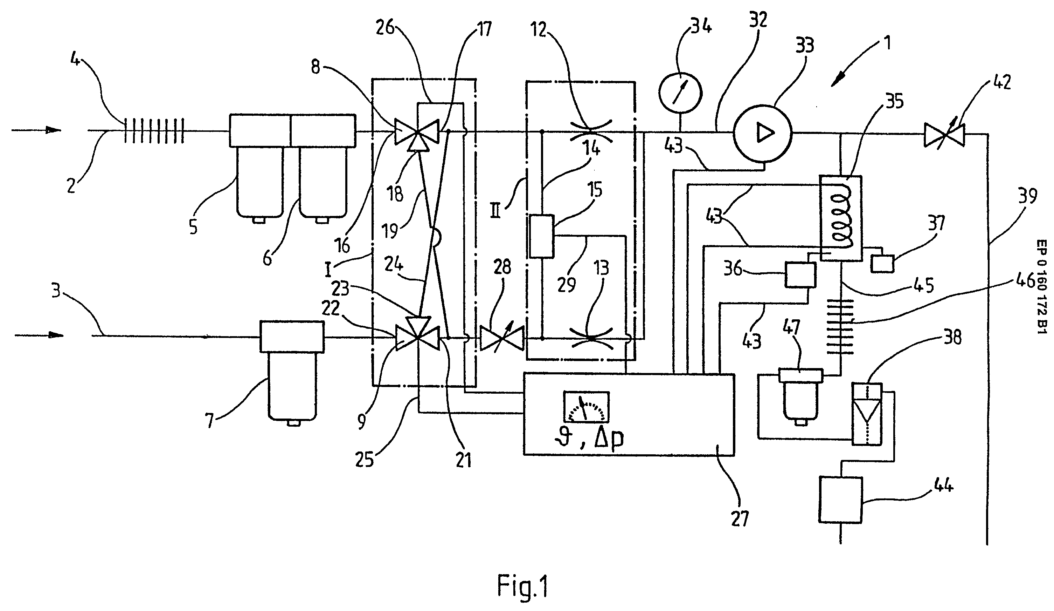

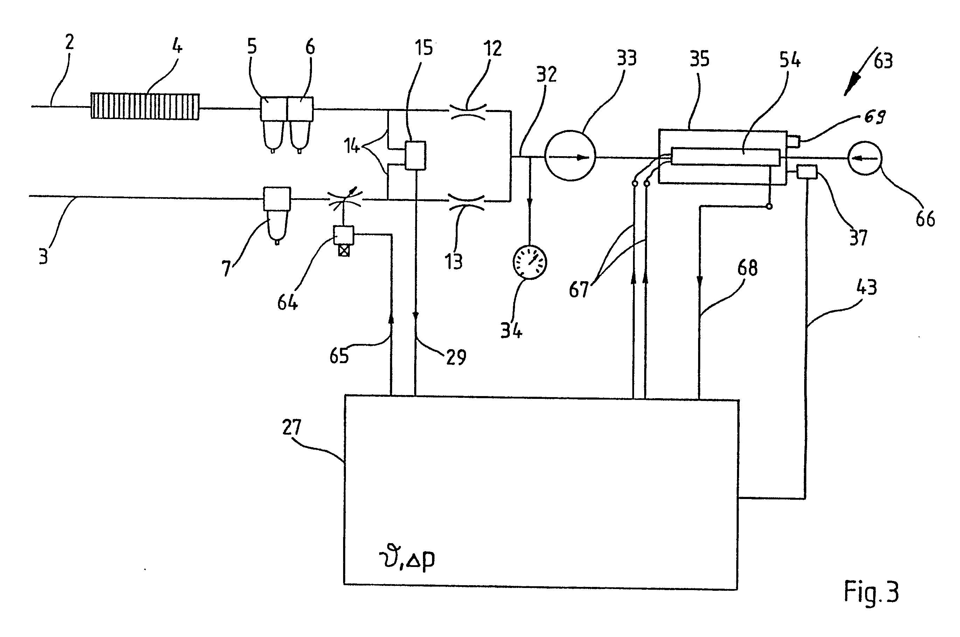

Das Flußschema einer Meßvorrichtung 1 enthält eine Abgasleitung 2, die beispielsweise in das Auspuffrohr eines Kraftfahrzeuges einmündet, sowie eine parallel dazu angeordnete Luftleitung 3 für einen Luft- oder Sauerstoffstrom. Die heißen Abgase durchströmen zunächst einen Kühler 4 und daran anschließend zum Ausfiltern der festen und flüssigen Bestandteile einen Wasserabscheider 5 und einen Feinfilter 6. Die zusätzliche Luft passiert hingegen zum Abscheiden etwaiger Verunreinigungen lediglich einen Feinfilter 7. Danach gelangen beide Förderströme zu einer entweder aus je einem Drei-Zwei-Wege-Magnetventil oder je einer Dosierdüse 12, 13 in jeder Leitung 2, 3 angeordneten Mischvorrichtung I oder II, die der Einfachheit halber beide in der Fig. 1 enthalten und strichpunktiert umrahmt dargestellt sind. Tatsächlich wird aber immer – wie anhand der Fig. 3 für eine Mischvorrichtung II mit Dosierdüsen noch erläutert werden wird – entweder nur die Mischvorrichtung I oder II eingesetzt werden, wobei den Dosierdüsen 12, 13 der Mischvorrichtung II in einer mit der Abgas- und Luftleitung 2, 3 verbundenen Zwischenleitung 14 noch ein Differenzdruckfühler 15 vorgeordnet ist.

Mit der Mischvorrichtung I läßt sich das konstante Mischverhältnis bzw. das Kalibrieren des Verdünnungsgrades durch ein Kreuzen des Abgas- und des Luftstromes mittels der beiden Drei-Zwei-Wege-Magnetventile 8, 9 erreichen. Das in der Abgasleitung 2 mit einem Ventileingang 16 und einem Ventilausgang 17 angeordnete Magnetventil 8 weist dazu einen dritten Anschluß 18 auf, der über eine Leitung 19 mit der hinter dem Ventilausgang 21 des Magnetventils 9 weitergeführten Luftleitung 3 verbunden ist. Das Magnetventil 9 der Luftleitung 3 ist in gleicher Weise angeordnet, d.h. neben einem Ventileingang 22 ist ein dritter Anschluß 23 über eine Leitung 24 mit der hinter dem Ventilausgang 17 des Magnetventils 8 fortgeführten Abgasleitung 2 verbunden.

Von den Magnetventilen 8, 9 gehen dünngezogen dargestellte Steuerleitungen 25, 26 zu einer zentralen Schalt-, Steuer- und Anzeigeeinheit 27, so daß sich das gewünschte Mischverhältnis durch Verstellen eines in der Luftleitung 3 hinter dem Magnetventil 9 angeordneten Drosselventils 28 erreichen läßt. Für das Mischverhältnis bzw. den Verdünnungsgrad liegt nämlich eine feste Zuordnung zwischen der Sauerstoff- oder Kohlendioxyd-Konzentrationsanzeige vor und nach dem Kreuzen/Mischen der Gasströme vor. Bei Abweichungen von dieser festen Zuordnung läßt sich über die Steuereinheit 27 das Drosselventil 28 beeinflussen und bis zum Wiederherstellen des Mischungsverhältnisses entweder mehr oder weniger Zusatzluft in die Abgasleitung 2 einbringen.

Bei einem Betrieb mit der Mischvorrichtung II wird davon ausgegangen, daß die Temperatur des Abgases und der Zusatzluft durch die Art der Leitungsführung vor den beiden Dosierdüsen 12, 13 gleich ist. Wenn die Düsen überkritisch durchströmt werden, hängt der Gaszustand dann nur mehr vom Druck der beiden Gasströme ab, den der Differenzdruckfühler 15 vor den beiden Dosierdüsen 12, 13 erfaßt. Bei Abweichungen geht vom Differenzdruckfühler 15 über eine Steuerleitung 29 ein Signal zur Steuereinheit 27, worauf wiederum das Drosselventil 28 betätigt und unterschiedliche Druckverluste bis zum Wiederherstellen eines vorgegebenen Differenzdruckes und damit des konstanten Mischungsverhältnisses ausgeglichen werden.

Beide Gasströme vereinigen sich nach dem Verlassen der Mischvorrichtung I oder II in einer gemeinsamen Ausgangsleitung 32, in der zum Ansaugen der beiden Förderströme eine vorzugsweise als Vakuumpumpe 33 ausgebildete Pumpe sowie ein Betriebsdruckanzeiger 34, z.B. ein Unterdrucksensor, angeschlossen wird. Hinter der Vakuumpumpe 33 führt die Ausgangsleitung 32 zu einem als elektrisch beheizter Konverter ausgeführten Nachbrenner 35, dem ein Temperaturfühler 36, eine Sauerstoffsonde 37, ein Durchflußmesser 38 sowie in einer Nebenleitung 39 ein Bypassventil 42 zugeordnet sind. Die Pumpe 33, sowie am ein- und austrittsseitigen Ende der Nachbrenner 35 und der Temperaturfühler 36 sind über Steuer- bzw. Signalleitungen 43 an die Schalt-, Steuer- und Anzeigeeinheit 27 angeschlossen.

Für den Fall, daß keine Sauerstoff-Messung sondern eine Kohlendioxyd-Messung durchgeführt wird, entfällt die Sauerstoffsonde 37 im Nachbrenner 35 und ist ein unmittelbares Messen nicht möglich. In diesem Fall befindet sich in einer zu einem separaten Gassensor 44 führenden Leitung 45 ein Kühler 46 mit einem nachgeschalteten Wasserabscheider 47, der den bei der Nachreaktion der unverbrannten Kohlenwasserstoffe entstehenden Wasserdampf vor dem Messen des Kohlendioxydanteils entfernt. Unabhängig davon, ob die Luftzahl direkt im Nachbrenner 35 mittels der Sauerstoffsonde 37 oder in dem nachgeschalteten separaten Gassensor 44 ermittelt wird, gelangt ein Signal zu der zentralen Schalt- und Steuereinheit 27, wo der festgestellte Wert angezeigt wird.

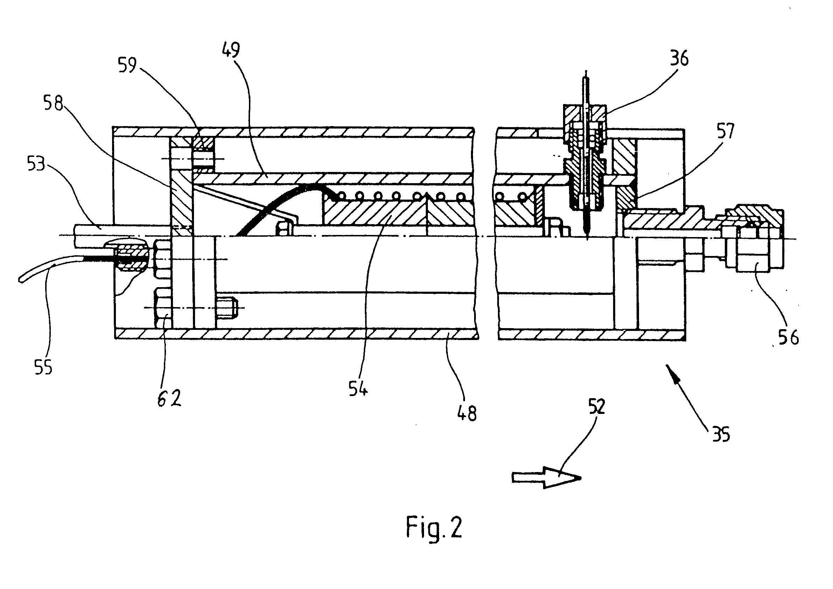

Der Nachbrenner 35 gemäß Fig. 2 besteht aus einem äußeren zylindrischen Gehäuse 48 und einem inneren zylindrischen Gehäuse 49. Der zur Luftzahlermittlung benötigte Meßstrom gelangt in der durch den Pfeil 52 angegebenen Strömungsrichtung über das an die Leitung 32 angeschlossene Anschlußstück 53 in das innere Gehäuse 49, wo das Abgas-/Luftgemisch aufbereitet, d.h. bis zur völligen Oxydation verbrannt wird. Die dazu erforderliche Verbrennungstemperatur liefert eine im inneren Gehäuse 49 lagesicher angeordnete Heizwicklung 54, die sich mittels einer hitzebeständigen Zuleitung 55 an eine nicht dargestellte Stromquelle anschließen läßt.

Der Meßstrom, insbesondere eine Teilmenge mit geringem Volumen, läßt sich bereits mit einer Heizleistung von ca. 80 Watt auf Temperaturen von über 700 °C aufheizen und bei einer Verweilzeit von mindestens 200 ms nachverbrennen. Der von außen in den Nachbrenner 35 ragende Temperaturfühler 36 durchdringt die Gehäuse 48, 49 und mißt und regelt die Temperatur des Gasstroms im inneren Gehäuse 49. Bei Erreichen der erforderlichen Temperatur signalisiert der Fühler 36 die Betriebsbereitschaft, so daß das aufbereitete Gas über das austrittsseitige, mit der Leitung 45 verbundene Anschlußstück 56 abströmen kann. Das Anschlußstück 56 ist im übrigen in eine ausgangsseitig mit dem inneren Rohrgehäuse 49 verschweißte Abschlußplatte 57 eingeschraubt. An der Eingangsseite des Nachbrenners 35 ist das innere Gehäuse 49 von einer Platte 58 abgeschlossen, deren Außendurchmesser dem Innendurchmesser des äußeren Gehäuses 48 entspricht, so daß sich das die elektrische Heizeinrichtung 54 aufnehmende innere Gehäuse 49 mit der Platte 58 bis gegen nach innen vorspringende Anschläge 59 des äußeren Gehäuses 48 einschieben und mittels umfangsverteilt angeordneten Schrauben 62 darin festlegen läßt.

Die in Fig. 3 mit einer im Rahmen der Erfindung bevorzugten Mischvorrichtung mittels Dosierdüsen 12, 13 dargestellte Meßeinheit 63 unterscheidet sich von der Meßeinheit gemäß Fig. 1 durch das mit dem Differenzdruckfühler 15 vor den Dosierdüsen 12, 13 gekoppelte, in der Luftleitung 3 angeordnete, motorbetriebene Regelventil 64, das mit einer Steuerleitung 65 an die zentrale Anzeige- und Steuereinheit 27 angeschlossen ist. Weiterhin ist dem Nachbrenner 35, der den gesamten, über die Leitung zugeführten Luft-/Abgas-Mischstrom mißt, so daß das Bypassventil 42 und die Nebenleitung 39 (vgl. Fig. 1) nicht benötigt werden, eine in der Nähe des Sensors 37 angeordnete, vorzugsweise in der ausgangsseitigen Gehäusewand befestigte Referenzluftpumpe 66 zugeordnet, die den Sauerstoffsensor 37 mit Zusatzluft spült und damit für ein zum Messen benötigtes ausgeglichenes Temperaturniveau sorgt und Drifterscheinungen des Sensors verhindert. Die Temperaturabweichungen werden auch noch dadurch begrenzt, daß von der Wicklung der Heizeinrichtung 54 als auch vom Sensor 37 des Nachbrenners 35 zusätzliche Steuerleitungen 67 bzw. 68 zur rechentechnischen Verarbeitung der gewonnenen Signale in die Anzeigeeinheit 27 führen, so daß sowohl Spannungsunterschiede bei der Regelung der Heizwicklung, als auch temperaturbedingte Meßungenauigkeiten des Sensors berücksichtigt werden können. Für ein ausgeglichenes Temperaturniveau sorgt weiterhin ein am Nachbrennergehäuse befestigter, schematisch dargestellter Ventilator 69, der einen Wärmestau verhindert, so daß sich ein Überhitzen der Elektronik vermeiden läßt.

**Patentansprüche**

1. Einkomponenten-Abgasmeßverfahren zum Bestimmen der Luftzahl bzw. Gemischzusammensetzung insbesondere von Ottomotoren, bei dem der Abgasstrom gekühlt, vom Kondensat befreit sowie gereinigt und anschließend mit einem gereinigten Luftstrom in einem konstanten Verhältnis gemischt wird, dadurch gekennzeichnet, daß die beiden Ströme druck- oder mengenabhängig sowie ventilgesteuert/-geregelt miteinander vermischt werden, die Konzentration der Meßwert-/Abgaskomponenten nach dem Mischen in Relation zu einem für jeden Strom vor dem Mischen feststehenden Ist-Wert gesetzt und bei Abweichungen vom Ist-Wert entweder ein Druckunterschied zwischen den beiden Strömen ausgeglichen oder die zugeführte Menge eines Stromes geändert und das Gemisch vollständig oxydiert und der Gehalt der zu bestimmenden Meßkomponenten in dem vollständig oxydierten Abgas-/Luftgemisch ermittelt wird.

2. Verfahren nach Anspruch 1, dadurch gekennzeichnet, daß eine kleine Teilmenge des Gemischs vollständig oxydiert und analysiert wird.

3. Verfahren nach Anspruch 1 oder 2, dadurch gekennzeichnet, daß zum Mischen eine jedem Strom zugeordnete Düse überkritisch durchströmt wird.

4. Vorrichtung zum Durchführen des Verfahrens nach Anspruch 1 oder 2, dadurch gekennzeichnet, daß in einer Abgasleitung (2) ein Kühler (4), ein Wasserabscheider (5) und ein Feinfilter (6) sowie in einer Luftleitung (3) ein Feinfilter (7) angeordnet sind, daß sich daran in jeder Leitung (2, 3) eine Dosierdüse (12, 13) anschließt, die mit einer gemeinsamen, zu einem nachgeschalteten Verbrenner (35) und einem Gassensor (44) oder einer Sauerstoffsonde (37) führenden Ausgangsleitung (32) verbunden ist und daß ein Differenzdruckfühler (15) mittels einer den Dosierdüsen (12, 13) vorgeordneten Zwischenleitung (14) an die Luft- und die Abgasleitung (2, 3) angeschlossen und mit einem gesteuerten/geregelten Drosselventil (28) in einer Leitung gekoppelt ist.

5. Vorrichtung nach Anspruch 4, gekennzeichnet durch ein in der Luftleitung (3) angeordnetes Drosselventil (28).

6. Vorrichtung zum Durchführen des Verfahrens nach Anspruch 1 oder 2, dadurch gekennzeichnet, daß der Differenzdruckfühler (15) vor den Dosierdüsen (12, 13) mit einem in der Luftleitung (3) angeordneten, motorbetriebenen Ventil (64) gekoppelt ist.

7. Vorrichtung nach einem oder mehreren der Ansprüche 4 bis 6, gekennzeichnet durch eine Vakuumpumpe (33) in der gemeinsamen Ausgangsleitung (32).

8. Vorrichtung zum Durchführen des Verfahrens nach Anspruch 1 oder 2, dadurch gekennzeichnet, daß in einer Abgasleitung (2) ein Kühler (4), ein Wasserabscheider (5) und ein Feinfilter (6) sowie in einer Luftleitung (3) ein Feinfilter (7) angeordnet sind, daß sich daran in jeder Leitung (2, 3) ein Drei-Zwei-Wege-Magnetventil (8, 9) anschließt, das mit einer gemeinsamen, zu einem nachgeschalteten Verbrenner (35) und einem Gassensor (44) oder einer Sauerstoffsonde (37) führenden Ausgangsleitung (32) verbunden ist, daß jedes Magnetventil (8, 9) mittels einer Zweigleitung (19, 24) am Ventilausgang (17, 21) des jeweils anderen Magnetventils mit der Luft- bzw. Abgasleitung (2 bzw. 3) verbunden und einem Magnetventil ein Drossel- oder Motorventil (28, 64) zugeordnet ist.

9. Vorrichtung nach Anspruch 8, gekennzeichnet durch ein Drossel- oder Motorventil (28, 64) in der Luftleitung (3).

10. Vorrichtung nach einem oder mehreren der Ansprüche 4 bis 9, gekennzeichnet durch einen als elektrisch beheizter Konverter ausgebildeten Nachbrenner (35).

11. Vorrichtung nach einem oder mehreren der Ansprüche 4 bis 10, gekennzeichnet durch einen im Nachbrenner (35) angeordneten Temperaturfühler (36).

12. Vorrichtung nach einem oder mehreren der Ansprüche 4 bis 11, dadurch gekennzeichnet, daß dem Nachbrenner (35) ein Durchflußmesser (38) und in einer Nebenleitung (39) ein Bypassventil (42) zugeordnet sind.

13. Vorrichtung nach einem oder mehreren der Ansprüche 4 bis 12, dadurch gekennzeichnet, daß zwischen dem Nachbrenner (35) und einem Gassensor (44) ein Kühler (46) und ein Wasserabscheider (47) angeordnet sind.

14. Vorrichtung nach einem oder mehreren der Ansprüche 4 bis 12, gekennzeichnet durch eine in den Nachbrenner (35) ragende Sauerstoffsonde (37).

15. Vorrichtung nach Anspruch 14, gekennzeichnet durch einen Zirkoniumdioxyd-Sauerstoffsensor (37).

16. Vorrichtung nach einem oder mehreren der Ansprüche 4 bis 15, gekennzeichnet durch einen am Nachbrenner (35) angeordneten Ventilator (66).

17. Vorrichtung nach einem oder mehreren der Ansprüche 4 bis 12 und 14 bis 16, dadurch gekennzeichnet, daß die Sauerstoffsonde (37) im Strömungsbereich des nachverbrannten Gemischstroms mißt.

18. Vorrichtung nach einem oder mehreren der Ansprüche 4 bis 13, gekennzeichnet durch einen mit dem Gassensor (44) gekoppelten Temperaturfühler (36).

19. Vorrichtung nach einem oder mehreren der Ansprüche 4 bis 18, dadurch gekennzeichnet, daß das Gehäuse (49) des Nachbrenners (35) aus Keramik besteht und eine isolierende Umhüllung aufweist.

## Claims

1. A single component exhaust gas measuring process for determining the air number (lambda value) or mixture composition, in particular of Otto engines, in which the exhaust gas stream is cooled, freed from condensate and purified and is then mixed in a constant proportion with a purified stream of air, characterised in that the two streams are mixed together in a manner dependent on pressure or quantity and valve-controlled or regulated, the concentration of the measured values/exhaust gas components after the mixing is set in relation to an actual value fixed for each stream before the mixing and, in case of deviation from the actual value, either a pressure difference between the two streams is equalised or the amount of one stream supplied is changed and the mixture is completely oxidised and the content of the components to be measured in the completely oxidised exhaust gas/air mixture is determined.

2. A process according to claim 1, characterised in that a small part of the mixture is completely oxidised and analysed.

3. A process according to claim 1 or claim 2, characterised in that mixing is effected by supercritical flow through a nozzle associated with each stream.

4. Apparatus for carrying out the process according to claim 1 or 2, characterised in that a cooler (4), a water separator (5) and a fine-mesh filter (6) are arranged in an exhaust gas line (2) and a fine-mesh filter (7) is arranged in an air line (3), that in each line (2, 3) a metering nozzle (12, 13) follows that is connected to a common outlet line (32) leading to a following burner (35) and a gas sensor (44) or an oxygen probe (37), and that a differential pressure sensor (15) is connected to the air line and the gas line (2, 3) by means of an intermediate line (14) arranged before the metering nozzles (12, 13) and is coupled in a line with a controlled/regulated throttle valve (28).

5. Apparatus according to claim 4, characterised by a throttle valve (28) arranged in the air line (3).

6. Apparatus for carrying out the process according to claim 1 or claim 2, characterised in that the differential pressure sensor (15) is coupled before the metering nozzles (12, 13) with a motor-operated valve (64) arranged in the air line (3).

7. Apparatus according to one or more of claims 4 to 6, characterised by a vacuum pump (33) in the common outlet line (32).

8. Apparatus for carrying out the process according to claim 1 or claim 2, characterised in that a cooler (4), a water separator (5) and a fine-mesh filter (6) are arranged in an exhaust gas line (2) and a fine-mesh filter (7) is arranged in an air line (3), that a three-two-way magnetic valve (8, 9) is connected in each line (2, 3) and is connected to an outlet line (32) leading to a following burner (35) and a gas sensor (44) or an oxygen probe (37), that each magnetic valve (8, 9) is connected by a branch line (19, 24) at the valve outlet (17, 21) of the respective other magnetic valve to the air or exhaust gas line (2 or 3 respectively) and a throttle or motor valve (28, 64) is associated with one magnetic valve.

9. Apparatus according to claim 8, characterised by a throttle or motor valve (28, 64) in the air line (3).

10. Apparatus according to any one or more of claims 4 to 9, characterised by an afterburner (35) in the form of an electrically heated convertor.

11. Apparatus according to any one or more of claims 4 to 10, characterised by a temperature sensor (36) arranged in the afterburner (35).

12. Apparatus according to any one or more of claims 4 to 11, characterised in that a flow meter (38) and, in a by-pass line (39), a bypass valve (42), are associated with the afterburner (35).

13. Apparatus according to any one or more of claims 4 to 12, characterised in that a cooler (46) and a water separator (47) are arranged between the afterburner (35) and a gas sensor (44).

14. Apparatus according to any one or more of claims 4 to 12, characterised by an oxygen probe (37) projecting into the afterburner (35).

15. Apparatus according to claim 14, characterised by a zirconium dioxide oxygen sensor (37).

16. Apparatus according to any one or more of claims 4 to 15, characterised by a fan (66) arranged on the afterburner (35).

17. Apparatus according to any one or more of claims 4 to 12 and 14 to 16, characterised in that the oxygen probe (37) measures in the region in which the afterburned mixture stream flows.

18. Apparatus according to any one or more of claims 4 to 13, characterised by a temperature sensor (36) coupled with the gas sensor (44).

19. Apparatus according to any one or more of claims 4 to 18, characterised in that the housing (49) of the afterburner (35) consists of ceramic and has an insulating jacket.

## Revendications

1. Procédé de mesure d'une composante de gaz d'échappement pour déterminer l'indice d'air ou la composition du mélange, en particulier de moteurs à allumage par étincelle, dans lequel le gaz d'échappement est refroidi, débarrassé de condensat et purifié, puis mélangé avec un courant d'air purifié dans un rapport constant, caractérisé en ce que l'on mélange ensemble les deux courants par la commande ou le réglage d'une soupape en fonction de la pression ou du débit, on détermine la concentration des composantes de valeur de mesure ou de gaz d'échappement après mélange en relation à une valeur effective établie pour chaque courant avant mélange et, en cas de déviations par rapport à la valeur effective, soit on équilibre une différence de pression entre les deux courants, soit on modifie le débit d'admission d'un courant, puis on oxyde complètement le mélange et on établit la valeur des composantes de mesure à déterminer dans le mélange de gaz d'échappement ou d'air complètement oxydé.

2. Procédé selon la revendication 1, caractérisé en ce qu'on oxyde complètement et on analyse une faible quantité partielle du mélange.

3. Procédé selon la revendication 1 ou 2, caractérisé en ce que, pour le mélange, une buse associée à chaque courant est traversée par un écoulement de façon surcritique.

4. Dispositif pour la mise en œuvre du procédé selon la revendication 1 ou 2, caractérisé en ce qu'il comprend un condenseur (4), un séparateur d'eau (5) et un filtre fin (6) montés dans une conduite de gaz d'échappement (2) et un filtre fin (7) monté dans une conduite d'air (3), en ce qu'une buse de réglage (12, 13) est raccordée sur chaque conduite (2, 3), laquelle buse est reliée à une conduite de sortie commune (32) conduisant à un brûleur (35) intercalé à la suite et à un détecteur de gaz (44) ou à une sonde d'oxygène (37) et en ce qu'un capteur de pression différentielle (15) est raccordé à la conduite d'air ou de gaz d'échappement (2, 3) au moyen d'une conduite intermédiaire (14) montée en avant de l'une des buses (12, 13) et est couplé à une soupape à papillon (28) commandée ou réglée dans une conduite.

5. Dispositif selon la revendication 4, caractérisé par une soupape à papillon (28) montée dans la conduite d'air (3).

6. Dispositif pour la mise en œuvre du procédé selon la revendication 1 ou 2, caractérisé en ce que le capteur de pression différentielle (15) est couplé en avant des buses de réglage (12, 13) à une soupape (24) actionnée par un moteur et disposée dans la conduite d'air (3).

7. Dispositif selon l'une ou plusieurs des revendications 4 à 6, caractérisé par une pompe à vide (33) dans la conduite de sortie commune (32).

8. Dispositif pour la mise en œuvre du procédé selon la revendication 1 ou 2, caractérisé en ce qu'il comprend un condenseur (4), un séparateur d'eau (5) et un filtre fin (6) montés dans une conduite de gaz d'échappement (2), ainsi qu'un filtre fin (7) monté dans une conduite d'air (3), en ce qu'une électrovanne à trois-deux voies (8, 9) est raccordée sur chaque conduite (2, 3), laquelle électrovanne est reliée à une conduite de sortie commune (32) conduisant à un brûleur (35) intercalé à la suite et à un détecteur de gaz (44) ou à une sonde d'oxygène (37), en ce que chaque électrovanne (8, 9) est reliée à la conduite d'air ou de gaz d'échappement (2, 3) par une conduite de dérivation (19, 24) prévue à la sortie d'électrovanne (17, 21) de l'autre électrovanne et en ce qu'une soupape à papillon ou à moteur (28, 64) est associée à une électrovanne.

9. Dispositif selon la revendication 8, caractérisé par une soupape à papillon ou à moteur (28, 64) dans la conduite d'air (3).

10. Dispositif selon l'une ou plusieurs des revendications 4 à 9, caractérisé par un post brûleur (35) réalisé sous la forme d'un convertisseur chauffé électriquement.

11. Dispositif selon l'une ou plusieurs des revendications 4 à 10, caractérisé par un capteur de température (36) prévu dans le post-brûleur (35).

12. Dispositif selon l'une ou plusieurs des revendications 4 à 11, caractérisé en ce qu'un débitmètre (38) et une soupape de dérivation (42) dans une conduite secondaire (39) sont associés au post-brûleur (35).

13. Dispositif selon l'une ou plusieurs des revendications 4 à 12, caractérisé en ce qu'un condenseur (46) et un séparateur d'eau (47) sont montés entre le post-brûleur (35) et un détecteur de gaz (44).

14. Dispositif selon l'une ou plusieurs des revendications 4 à 12, caractérisé par une sonde d'oxygène (37) dépassant dans le post-brûleur (35).

15. Dispositif selon la revendication 14, caractérisé par un détecteur d'oxygène (37) en dioxyde de zirconium.

16. Dispositif selon l'une ou plusieurs des revendications 4 à 15, caractérisé par un ventilateur (66) agencé sur le post-brûleur (35).

17. Dispositif selon l'une ou plusieurs des revendications 4 à 12 et 14 à 16, caractérisé en ce que la sonde d'oxygène (37) réalise la mesure dans la région d'écoulement du courant de mélange post-brûlé.

18. Dispositif selon l'une ou plusieurs des revendications 4 à 13, caractérisé par un capteur de température (36) couplé au détecteur de gaz (44).

19. Dispositif selon l'une ou plusieurs des revendications 4 à 18, caractérisé en ce que le boîtier (49) du post-brûleur (35) se compose de céramique et présente une enveloppe isolante.

Fig.1

Fig. 2

EP 0 160 172 B1

Fig.3

EP 0 160 172 B1